# EUROPÄISCHE PATENTSCHRIFT

(11) **EP 2 654 699 B1**
(45) Veröffentlichungstag und Bekanntmachung des Hinweises auf die Patenterteilung: **09.05.2018**
(21) Anmeldenummer: 11788170.6
(22) Anmeldetag: 28.11.2011
(51) Int. Cl.: A61K 8/41, A61K 8/44, A61K 8/49, A61K 8/60, A61Q 5/02, A61Q 5/12, A61Q 19/00, A61Q 19/10

(54) **WIRKSTOFFKOMBINATIONEN AUS GLUCOSYLGLYCERIDEN UND EINEM ODER MEHREREN KONSERVIERUNGSMITTELN**
ACTIVE INGREDIENT COMBINATIONS OF GLYCERYL GLUCOSIDES AND ONE OR MORE PRESERVATIVES
COMBINAISONS DE PRINCIPES ACTIFS CONSTITUÉES DE GLUCOSYLGLYCÉRIDES ET D'UN OU DE PLUSIEURS CONSERVATEURS

(30) Priorität: 23.12.2010 DE 102010055768
(43) Veröffentlichungstag der Anmeldung: 30.10.2013
(73) Patentinhaber: Beiersdorf AG, 20253 Hamburg (DE)
(72) Erfinder: KRÖPKE, Rainer, 22869 Schenefeld (DE); FRESE, Christian, 22765 Hamburg (DE); SCHERNER, Cathrin, 22844 Norderstedt (DE); GRIEBENOW, Martin, 22305 Hamburg (DE); MÖLLGAARD, Svenja Lena, 22337 Hamburg (DE)
(74) Vertreter: Wilke, Jochen
(86) Internationale Anmeldenummer: PCT/EP2011/071138
(87) Internationale Veröffentlichungsnummer: WO 2012/084410

(56) Entgegenhaltungen:
- EP-A1- 1 923 045
- DE-A1-102005 023 639
- DE-A1-102006 055 043
- JP-A- 2008 024 628
- US-A1- 2009 124 576

## Beschreibung

Die vorliegende Erfindung betrifft Wirkstoffkombinationen aus Glucosylglyceriden und einem oder mehreren Konservierungsmitteln sowie deren Verwendung auf dem Gebiete der kosmetischen sowie der pharmazeutischen Dermatologie.

Insbesondere betrifft die vorliegende Erfindung Wirkstoffe und kosmetische oder dermatologische Zubereitungen, solche Wirkstoffkombinationen enthaltend.

Die äußerste Schicht der Epidermis, das Stratum corneum (Hornschicht), ist als wichtige Barriereschicht von besonderer Bedeutung u.a. für den Schutz vor Umwelteinflüssen und Austrocknung. Die Hornschicht wird im Kontakt mit der Umwelt ständig abgenutzt und muß deshalb ununterbrochen erneuert werden.

Ein heute in der Fachwelt weitverbreitetes Hautmodell faßt das Stratum corneum als Zwei-Komponenten-System, ähnlich einer Ziegelsteinmauer (Ziegelstein-Mörtel-Modell), auf. In diesem Modell entsprechen die Korneozyten (Hornzellen) den Ziegelsteinen, die kompliziert zusammengesetzte Lipidmembran in den Interzellularräumen entspricht dem Mörtel.

Außer ihrer Barrierewirkung gegen externe chemische und physikalische Einflüsse tragen die epidermalen Lipide auch zum Zusammenhalt der Hornschicht bei und haben Einfluß auf die Hautglätte. Im Gegensatz zu den Talgdrüsenlipiden, die keinen geschlossenen Film auf der Haut ausbilden, sind die epidermalen Lipide über die gesamte Hornschicht verteilt.

Das äußerst komplexe Zusammenwirken der feuchtigkeitsbindenden Substanzen und der Lipide der oberen Hautschichten ist für die Regulation der Hautfeuchte sehr wichtig. Daher enthalten Kosmetika in der Regel, neben ausgewogenen Lipidabmischungen und Wasser, wasserbindende Substanzen. Hierzu zählen u.a. Polyole wie Glycerin, Sorbit und Xylit, ethoxylierte Polyole sowie hydrolysierte Proteine. Weitere Anwendung finden die im natürlichen Feuchthaltefaktor (sogenannter Natural Moisturizing Factor = NMF) enthaltenen Substanzen, wie z.B. Harnstoff, Kohlenhydrate (z. B. Glucose) und Aminosäuren (z. B. Serin). Diese Substanzen sind daher für die Pflegeleistung eines kosmetischen Produktes von besonderer Bedeutung, insbesondere auch aufgrund ihrer relativ guten Haut- und Schleimhautverträglichkeit.

Der Wunsch nach sauberer Haut ist wohl so alt wie die Menschheit, denn Schmutz, Schweiß und Reste abgestorbener Hautpartikel bieten den idealen Nährboden für Krankheitserreger und Parasiten aller Art. Die Lust an der Körperhygiene wurde stetig verstärkt, als in den 60er Jahren des 20. Jahrhunderts neben der "klassischen" Seife auch flüssige Reinigungsmittel mit neu entwickelten synthetischen Tensiden formuliert werden konnten. Baden und Duschen sind seitdem aus unserem täglichen Leben nicht mehr wegzudenken. Den Verbrauchern stehen heutzutage eine Vielzahl von Produkten für die Reinigung der verschiedenen Körperpartien zur Verfügung.

Reinigung bedeutet das Entfernen von (Umwelt-) Schmutz und bewirkt damit eine Erhöhung des psychischen und physischen Wohlbefindens. Die Reinigung der Oberfläche von Haut und Haaren ist ein sehr komplexer, von vielen Parametern abhängiger Vorgang. Zum einen sollen von außen kommende Substanzen wie beispielsweise Kohlenwasserstoffe oder anorganische Pigmente aus unterschiedlichsten Umfeldern sowie Rückstände von Kosmetika oder auch unerwünschte Mikroorganismen möglichst vollständig entfernt werden. Zum anderen sind körpereigene Ausscheidungen wie Schweiß, Sebum, Haut- und Haarschuppen ohne tiefgreifende Eingriffe in das physiologische Gleichgewicht abzuwaschen.

An die Konservierung kosmetischer Zubereitungen wird ein hoher Anspruch gestellt, da einerseits eine ausreichende Konservierung zum Schutz der Formulierung vor bakteriellem Befall gewährleistet sein muss, andererseits negative Einflüsse der Konservierungsmittel auf die Verträglichkeit, Stabilität und sensorischen Eigenschaften der Formulierung verhindert werden müssen. Die Konservierungsmittel müssen demnach wirksam, toxikologisch unbedenklich, gut hautverträglich, stabil formulierbar sowie preiswert herstellbar sein.

Eine Vielzahl an Konservierungsmitteln, wie Formaldehydabspalter, Parabene, Phenole- und deren Derivate, Bisguanidine und halogenierte Verbindungen, haben zwar eine hohe antimikrobielle Wirksamkeit, können aber Hautirritationen und allergische Reaktionen verursachen und werden daher von Verbrauchern kritisch betrachtet und überwiegend als unerwünscht angesehen.

Parabenfreie Konservierungsmittel, wie z.B. Isothiazolinone, Benzethoniumchlorid, Piroctonolamin und Lauroylethylarginat werden als Konservierungsmittel eingesetzt. Ihnen ist gemeinsam, dass sie auf der Haut bei der Verdünnung mit Wasser ein glitschiges Hautgefühl erzeugen und ein unbefriedigendes Viskositätsverhalten bei sehr niedrigen Temperaturen aufweisen. Unter diesen Umständen lassen sich derartige Zubereitungen nicht mehr der Verpackung entnehmen.

Es besteht daher der Bedarf an Zubereitungen, die die Nachteile des Standes der Technik beheben.

Es war nach all diesem überraschend und nicht vorhersehbar, daß die Verwendung kosmetischer Zubereitungen, umfassend eine Wasser und/oder Ölphase sowie ferner Wirkstoffkombinationen aus
(i) einem oder mehreren Konservierungsmitteln gewählt aus der Gruppe der Isothiazolinone, Benzethoniumchlorid, Piroctonolamin und Lauroylethylarginat
   und.
(ii) einem oder mehreren Glucosylglyceriden gewählt aus der Gruppe der der allgemeinen Formel 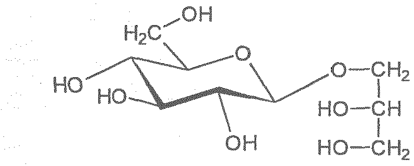 und/oder der allgemeinen Formel 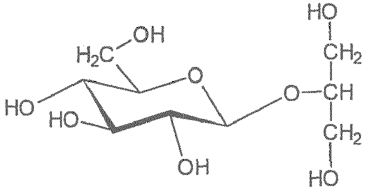 und/oder der allgemeinen Formel 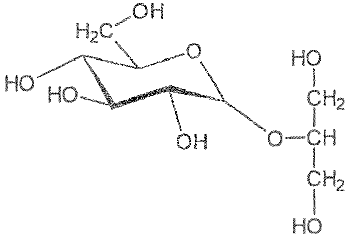 und/oder der allgemeinen Formel 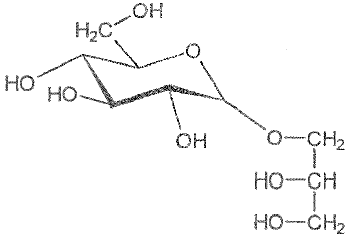 bei denen das oder die Glucosylglyceride in der Wasser- und/oder Ölphase in Konzentrationen von 0,001 - 40,00 Gew.-%, bevorzugt 0,005 - 15,00 Gew.-%, besonders bevorzugt 0,01 - 12,00 Gew.-%, jeweils bezogen auf das Gesamtgewicht der Zusammensetzung, vorliegen und bei denen das oder die mehreren Konservierungsmittel gewählt aus der Gruppe der Isothiazolinone, Benzethoniumchlorid, Piroctonolamin und Lauroylethylarginat in Konzentrationen von 0,00001 bis 10 Gew.-%, bes. bevorzugt 0,001-5 Gew.-%, bes. bevorzugt 0,005% - 3 Gew.-% jeweils bezogen auf das Gesamtgewicht der Zusammensetzung, vorliegt oder vorliegen, ferner enthaltend
(iii) eine Substanz, gewählt aus der Gruppe der anionischen, nichtionischen bzw. amphoteren Tenside und der kationischen Polymere
   als Reinigungsmittel,
   die Nachteile des Standes der Technik beseitigen.
Überraschend konnte die Aufgabe gelöst werden durch den Einsatz von Glycerylglucosiden, insbesondere in wäßrigen Reinigungsrezepturen enthaltend spezielle Konservierungsmittel. Diese Zubereitungen zeichnen sich durch eine hervorragende Sensorik und ein verbessertes Viskositätsverhältnis bei niedrigen & hohen Temperaturen aus.

Dabei wird die Zubereitung bevorzugt als Duschgel, Schaum- und Wannenbad, Shampoo und/oder Gesichtsreiniger verwendet. Kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe oder auch ein Aerosol darstellen.

Es ist erfindungsgemäß vorteilhaft, wenn die kosmetische Zubereitung in einer Flasche, Quetschflasche, Pumpspray-, oder Aerosoldose aufbewahrt und aus dieser heraus angewendet werden.

Die Zubereitung enthält vorteilhaft ein oder mehrere anionische Tenside. Diese liegen erfindungsgemäß vorteilhaft in einer Konzentration von 1 bis 20 Gewichts-%, und erfindungsgemäß bevorzugt in einer Konzentration von 5 bis 12 Gewichts-%, jeweils bezogen auf das Gesamtgewicht der Zubereitung, in der Zubereitung vor. Erfindungsgemäß besonders bevorzugt ist dabei der Einsatz von Nariumlaurylethersulfat als anionischem Tensid. Die Zubereitung enthält vorteilhaft ein oder mehrere amphotere Tenside. Diese liegen erfindungsgemäß vorteilhaft in einer Konzentration von 1 bis 20 Gewichts-%, und erfindungsgemäß bevorzugt in einer Konzentration von 3 bis 8 Gewichts-%, jeweils bezogen auf das Gesamtgewicht der Zubereitung, in der Zubereitung vor. Erfindungsgemäß besonders bevorzugt ist dabei der Einsatz von Cocamidopropylbetain als amphoterem Tensid.

Darüber hinaus kann die Zubereitung in vorteilhafter Weise auch nichtionische Tenside enthalten. Erfindungsgemäß besonders bevorzugt ist dabei der Einsatz von PEG-7 Glycerylcocoat und/oder PEG-40 hydriertem Rizinusöl als nichtionischem Tensid.

Es ist ebenfalls vorteilhaft, den Zubereitungen im Sinne der vorliegenden Erfindung kationische Polymere zuzufügen. Geeignete kationische Polymere sind beispielsweise
- quaternisierte Cellulose-Derivate, wie z.B. Polyquaternium-10, wie sie unter den Bezeichnungen Celquat und Polymer JR im Handel erhältlich sind
- kationische Guar-Derivate, wie insbesondere die unter den Handelsnamen Cosmedia Guar und Jaguar vertriebenen Produkte
- polymere Dimethyldiallylammoniumsalze und deren Copolymere mit Estern und Amiden von Acrylsäure und Methacrylsäure, wie insbesondere die unter den Bezeichnungen Merquat 100 und Merquat 550 im Handel erhältlichen Produkte.

Vorteilhaft ist es insbesondere, kationisches Polymer oder Mischungen aus kationischen Polymeren in einer Konzentration von 0.01 bis 2 Gewichts-%, bevorzugt in einer Konzentration von 0.05 bis 1.5 Gewichts-% und besonders bevorzugt von 0.1 bis 1.0 Gewichts-%, jeweils bezogen auf das Gesamtgewicht der Zubereitung, einzusetzen.

Die Zusammensetzungen enthalten außer den vorgenannten Substanzen gegebenenfalls die in der Kosmetik üblichen Zusatzstoffe, beispielsweise Parfüm, Farbstoffe, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, Pigmente, die eine färbende Wirkung haben, Verdickungsmittel, weichmachende, anfeuchtende und/oder feuchthaltende Substanzen, oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Die erfindungsgemäß vorteilhaften einsetzbaren Wirk-, Hilfs- und Zusatzstoffe sind dabei keineswegs auf die hier namentlich erwähnten Stoffe und Verbindungen beschränkt.

Als übliche kosmetische Trägerstoffe zur Herstellung der desodorierenden Zubereitungen gemäß der erfindungsgemäßen Verwendung können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z.B. Hydroxyethyl- oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosiät.

Es ist ebenfalls vorteilhaft, den Zubereitungen übliche Antioxidantien zuzufügen. Es können als günstige Antioxidantien alle für kosmetische und/oder dermatologische Anwendungen geeigneten oder gebräuchlichen Antioxidantien verwendet werden.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern die kosmetische oder dermatologische Zubereitung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte.
Insbesondere werden Gemische der vorstehend genannten Lösungsmittel verwendet. Bei alkoholischen Lösungsmitteln kann Wasser ein weiterer Bestandteil sein.

Als Treibmittel für aus Aerosolbehältern versprühbare kosmetische und/oder dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Kosmetische Zubereitungen können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Es folgen vorteilhafte Ausführungsbeispiele der vorliegenden Erfindung.

### Methode:

Die Messungen wurden unter folgenden Bedingungen durchgeführt:

| | |
|---|---|
| Gerät: | Ares 8 |
| Messung: | GEFR (rheologische Gefrierpunktbestimmung) |
| Programm: | dynamische Temperaturschritte von 25°C bis -30°C in 1°C-Schritten |
| Meßsystem: | Platte/Platte 25mm, 1mm Spalt |

### Beispielrezepturen

**Shampoozubereitungen**
alle Konzentrationsangaben in Gew.-%

| **Phase** | **INCI** | **1.1** | **1.2** | **1.3** | **1.4** |
|---|---|---|---|---|---|
| A | Wasser VES | ad 100 | ad 100 | ad 100 | ad 100 |
| A | Texapon N 70 | 10.50 | 12.00 | 6.50 | 13.00 |
| A | Rewopol SB CS 50 | - | 7.25 | - | - |
| A | Tego Betain F 50 | 11.50 | 9.50 | 13.00 | 10.00 |
| A | Merquat 550 | 2.50 | - | 2.50 | 2.50 |
| A | Panthenol | - | 0.50 | 1.00 | 0.10 |
| A | Methylisothiazolinone | 0.10 | 0.05 | 0.01 | 0.20 |
| A | Benzethoniumchlorid | 0.01 | 0.10 | 0.03 | 0.20 |
| A | Glyceryl Glucoside | 0.05 | 0.50 | 1.00 | 5.00 |
| B | Ucare Polymer JR 400 | 0.10 | 0.30 | 0.10 | - |
| B | Wasser VES | 15.00 | 15.00 | 15.00 | - |
| C | Wasser VES | - | 10.00 | 10.00 | - |
| C | Natronlauge 45%ig | - | 0.01 | 0.01 | - |
| C | Jaguar Excel | - | 0.10 | 0.12 | - |
| C | Zitronensäure | - | 0.03 | 0.03 | - |
| D | Eumulgin HRE 40 | 0.30 | 0.40 | 0.30 | 0.40 |
| D | Jojobaöl | 0.01 | 0.01 | 0.01 | 0.01 |
| D | Parfum | 0.60 | 0.80 | 0.90 | 0.70 |
| E₁ | Euperlan PK 900 | 4.00 | 6.00 | - | - |
| E₂ | Natriumchlorid* | 2.20 | 1.80 | 1.80 | 1.30 |
| E₃ | Zitronensäure** | 0.15 | 0.10 | 0.40 | 0.22 |

| | | | | | |
|---|---|---|---|---|---|
| * Menge variabel zur Einstellung einer Viskosität im Bereich von 3000 - 4500 mPas (gemssen mit HAAKE viscotester VT02 mit Rotor 1) ** Menge variabel zur Einstellung eines pH-Wertes von 4,8 bis 5,8 | | | | | |

Die Inhaltsstoffe der Phase A werden miteinander vermischt, bis eine homogene Phase entsteht.

Ucare Polymer JR 400 wird in das Wasser der Phase B eingestreut. Die Phase B wird unter Rühren auf ca. 70°C erwärmt bis eine klare Lösung entsteht. Phase B wird gekühlt und zur Phase A gegeben.

Das Wasser der Phase C wird mit der Natronlauge gemischt. Jaguar Excel wird unter Rühren langsam eingestreut. Anschließend wird die Mischung auf 70°C erwärmt und gerührt, bis eine homogene Phase entstanden ist. Unter Rühren wird die Zitronensäure zugegeben. Die Phase C wird abgekühlt und zur Phase A gegeben.

Eumulgin HRE 40 wird bei einer Temperatur von ca. 40°C aufgeschmolzen. Die übrigen Rohstoffe der Phase D werden zum Eumulgin HRE 40 gegeben. Die Phase wird homogen vermischt und zur Phase A gegeben.

Die Zugabe der Inhaltsstoffe der Phase E erfolgt in der angegebenen Reihenfolge. Es wird gerührt bis ein homogenes Shampoo entsteht.

**Duschgelzubereitungen**
alle Konzentrationsangaben in Gew.-%

| **Phase** | **INCI** | **2.1** | **2.2** | **2.3** | **2.4** | **2.5** |
|---|---|---|---|---|---|---|
| A | Wasser VES | ad 100 | ad 100 | ad 100 | ad 100 | ad 100 |
| A | Texapon N70 | 9.00 | 11.00 | 7.00 | 4.00 | 6.50 |
| A | Piroctone Olamine | 0.10 | 0.05 | 0.20 | 0.20 | 0.01 |
| A | Benzethoniumchlorid | 0.20 | - | 0.02 | - | 0.10 |
| A | Zitronensäure* | 0.35 | 0.35 | 0.35 | 0.35 | 0.50 |
| A | Rewoderm LI 520-70 ** | 0.10 | 0.10 | 0.10 | 0.10 | |
| A | Glucamate DOE-120 | - | - | - | - | 0.03 |
| A | Glycerin | - | - | 1.00 | - | 1.00 |
| A | Uvinul MS-40 | - | - | - | 0.50 | - |
| A | Glycerylglucosid | 0.01 | 0.10 | 3.00 | 1.00 | 1.00 |
| B | Eumulgin HRE 40 | 0.50 | 0.50 | 0.50 | 0.50 | 0.25 |
| B | Mandelöl | 0.01 | 0.01 | - | - | 0.20 |
| B | Sonnenblumenöl | - | - | 0.01 | 0.04 | - |
| B | Avocadoöl | - | - | - | 0.02 | - |
| B | Cetiol HE | 1.75 | 2.00 | 2.50 | 2.00 | 1.00 |
| B | Polyox WSR-301 | - | - | | 0.10 | |
| B | Parfum | 1.00 | 1.00 | 1.00 | 1.00 | 0.20 |
| C₁ | Merquat 550 | 3.00 | 6.00 | 4.00 | 5.00 | - |
| C₂ | Tego Betain F 50 | 15.00 | 20.00 | 12.00 | 25.00 | 15.00 |
| C₃ | Opulyn 301 | 1,00 | 1,00 | 1,00 | - | - |
| C₄ | Euperlan PK 900 | - | - | - | 4.00 | - |
| C₅ | Euperlan PK 771 | - | - | - | - | 4.00 |

| | | | | | | |
|---|---|---|---|---|---|---|
| * Menge variabel zur Einstellung eines pH-Wertes von 4,8 bis 5,3 ** Menge variabel zur Einstellung einer Viskosität im Bereich von 3000 - 5000 mPas (gemssen mit HAAKE viscotester VT02 mit Rotor 1) | | | | | | |

Die Inhaltsstoffe der Phase A werden vermischt, bis eine homogene Mischung entsteht.

Eumulgin HRE 40 wird bei einer Temperatur von ca. 40°C aufgeschmolzen. Die übrigen Rohstoffe der Phase B werden zum Eumulgin HRE 40 gegeben. Die Phase wird homogen vermischt und zur Phase A gegeben.

Die Zugabe der Inhaltsstoffe der Phase C erfolgt unter Rühren in der angegebenen Reihenfolge.

### W/O-Emulsionen

| | **1** | **2** | **3** | **4** | **5** |
|---|---|---|---|---|---|
| Triglycerindiisostearat | 1,0 | 0,5 | 0,25 | 2,0 | 3,0 |
| Diglycerindipolyhydroxystearat | 1,0 | 1,5 | 1,75 | 3,0 | 2,0 |
| Paraffinöl | 12,5 | 10,0 | 8,0 | 5,0 | 11,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Decyloleat | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Glycerylglucosid | 7,5 | 12,5 | 2,5 | 5 | 20 |
| Magnesiumsulfat | 0,5 | 0,6 | 0,5 | 0,7 | 1,0 |
| Glycerin | 3,0 | 5,0 | 10,0 | 15,0 | 1,5 |
| Parfum | q,s, | q,s, | q,s, | q,s, | q,s, |
| Ethanol | 2,0 | --- | 5,0 | --- | --- |
| Capryl-/Caprinsäuretriglycerid | 2,0 | 2,5 | 3,0 | 5,0 | 0,5 |
| Methylisothiazolinone | --- | 0,10 | --- | 0,06 | --- |
| Benzethoniumchlorid | 2,0 | --- | --- | 0,15 | --- |
| Piroctonolamin | --- | --- | 0,25 | 0,15 | --- |
| Lauroylethylarginat | --- | --- | --- | 0,15 | 1,75 |
| Wasser | ad 100 | ad 100 | ad 100 | ad 100 | ad 100 |

### W/O-Emulsionen

| | **6** | **7** | **8** | **9** | **10** |
|---|---|---|---|---|---|
| PEG-30 Dipolyhydroxystearat | --- | 0,5 | 0,25 | --- | 3,0 |
| Lanolin Alcohol | 1,0 | 1,5 | 1,75 | 3,0 | --- |
| Paraffinöl | 12,5 | 10,0 | 8,0 | 5,0 | 10,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Hydriertes Polyisobuten | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Microcrystalline Cellulose | 0,5 | 1,0 | 0,75 | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Parfum | q,s, | q,s, | q,s, | q,s, | q,s, |
| 1,3 Butylenglykol | 2,0 | --- | 5,0 | --- | --- |
| Glycerylglucosid | 2,0 | 2,5 | 3,0 | 5,0 | 0,5 |
| Methylisothiazolinone | --- | 0,10 | --- | 0,06 | --- |
| Benzethoniumchlorid | 2,0 | --- | --- | 0,15 | --- |
| Piroctonolamin | --- | --- | 0,25 | 0,15 | --- |
| Lauroylethylarginat | --- | --- | --- | 0,15 | 1,75 |
| Talkum | --- | --- | 0,05 | --- | 0,1 |
| Wasser | ad 100 | ad 100 | ad 100 | ad 100 | ad 100 |

### W/S-Emulsion

| | **11** | **12** | **13** | **14** | **15** |
|---|---|---|---|---|---|
| Cetyl PEG/PPG-10/1 Dimethicone | 1,0 | --- | -- | 3,0 | 5,0 |
| Cylomethicon + PEG/PPG-18/18 Dimethicon (90:10) | 10,0 | 12,5 | 25 | -- | -- |
| cyclisches Silikonöl (Cyclomethicon) | 12,5 | 15 | 22,0 | 20,0 | 15,5 |
| lineares Silikonöl (Dimethicon) | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Natriumcitrat | 1,0 | 0,1 | 0,4 | 0,9 | 2,5 |
| Parfum | q,s, | q,s, | q,s, | q,s, | q,s, |
| Glycerylglucosid | 10,0 | 15,0 | 7,5 | 5,0 | 2,5 |
| Methylisothiazolinone | --- | 0,10 | --- | 0,06 | --- |
| Benzethoniumchlorid | 2,0 | --- | --- | 0,15 | --- |
| Piroctonolamin | --- | --- | 0,25 | 0,15 | --- |
| Lauroylethylarginat | --- | --- | --- | 0,15 | 1,75 |
| modifizierte Stärke | --- | 2,5 | --- | 0,15 | -- |
| Wasser | ad 100 | ad 100 | ad 100 | ad 100 | ad 100 |

### W/S-Emulsionen

| | **16** | **17** | **18** | **19** | **20** |
|---|---|---|---|---|---|
| Cetyl PEG/PPG-10/1 Dimethicone | 1,0 | --- | -- | 3,0 | 5,0 |
| Cylomethicon + PEG/PPG-18/18 Dimethicon (90:10) | 10,0 | 12,5 | 25 | -- | -- |
| cyclisches Silikonöl (Cyclomethicon) | 12,5 | 15 | 8,0 | 20,0 | 17,5 |
| lineares Silikonöl (Dimethicon) | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |
| Glycerylglucosid | 3,0 | 5,0 | 10,0 | 15,0 | 1,5 |
| Milchsäure | 0,2 | 0,1 | 0,2 | --- | --- |
| Natriumlactat | 0,2 | 1,0 | 0.05 | --- | --- |
| Parfum | q,s, | q,s, | q,s, | q,s, | q,s, |
| Glycerin | 1,0 | 0,1 | 1,5 | 2,5 | 0,1 |
| Stearyldimethicon | 0,5 | --- | 0,7 | --- | --- |
| Methylisothiazolinone | --- | 0,10 | --- | 0,06 | --- |
| Benzethoniumchlorid | 2,0 | --- | --- | 0,15 | --- |
| Piroctonolamin | --- | --- | 0,25 | 0,15 | --- |
| Lauroylethylarginat | --- | --- | --- | 0,15 | 1,75 |
| modifizierte Stärke | --- | 2,5 | --- | 0,15 | -- |
| Wasser | ad 100 | ad 100 | ad 100 | ad 100 | ad 100 |

### Silikon in Wasser-Emulsion

| | **21** | **22** | **23** | **24** | **25** |
|---|---|---|---|---|---|
| Bis-PEG/PPG-16/16 PEG/PPG16/16 Dimethicone, Caprylic/Caprictriglycerid | 1,0 | 2,0 | 8,0 | 3,0 | 5,0 |
| cyclisches Silikonöl (Cyclomethicon) | 12,5 | 15 | 25,0 | 10,0 | 7,5 |
| lineares Silikonöl (Dimethicon) | 5,0 | 15,0 | 5,0 | 12,0 | 15,0 |
| Octyldodecanol | 0,5 | 1,0 | 0,75 | 3,0 | 0,25 |
| Glycerin | 5,0 | 7,5 | 10,0 | 3,0 | 1,0 |
| Panthenol | 0,5 | 1,0 | 0,75 | 0,25 | 0,1 |
| Parfum | q,s, | q,s, | q,s, | q,s, | q,s, |
| Stearyldimethicon | 0,5 | --- | 0,7 | --- | --- |
| Methylisothiazolinone | --- | 0,10 | --- | 0,06 | --- |
| Benzethoniumchlorid | 2,0 | --- | --- | 0,15 | --- |
| Piroctonolamin | --- | --- | 0,25 | 0,15 | --- |
| Lauroylethylarginat | --- | --- | --- | 0,15 | 1,75 |
| Glycerylglucosid | 5,0 | 2,5 | 7,5 | 15,0 | 12,5 |
| Wasser | ad 100 | ad 100 | ad 100 | ad 100 | ad 100 |

### O/W-Emulsion

| | **26** | **27** | **28** | **29** | **30** |
|---|---|---|---|---|---|
| Glycerylsteratcitrat | 1,0 | 1,5 | 1,5 | 0,5 | 0,25 |
| Cetylstearylalkohol | 2,5 | 5 | 0,5 | 2,0 | 1,5 |
| Ethylhexylmethoxycinnamat | 5,0 | 3,0 | 2,0 | 2,0 | 5,0 |
| Ethylhexylglycerin | 1 | --- | 0,5 | 1 | --- |
| Aluminiumchlorhydrat | --- | 1 | 0,02 | 1 | --- |
| Ethanol | --- | --- | 3,0 | 1 | -- |
| Ammonium Acryloyldimethyltairat/VP Copolymer | 0,5 | 0,75 | 0,5 | 2,0 | 0,25 |
| Blauer, wasserlöslicher Farbstoff (Blue No. | 0,5 | 1,0 | 0,4 | 3,0 | 0,25 |
| Carbomer | --- | --- | 0,2 | --- | 0,1 |
| Glycerylglucosid | 5,0 | 7,5 | 10,0 | 2,5 | 2,5 |
| Octyldodecanol | 0,5 | --- | 1,0 | --- | 2,0 |
| Caprylic/Capric Triglycerid | --- | --- | 1,0 | --- | 0,5 |
| Myristylmyristat | --- | 1,5 | 1,0 | 2,5 | --- |
| Parfum | q,s, | q,s, | q,s, | q,s, | q,s, |
| Meeressalz | --- | --- | 0,01 | 0,5 | --- |
| Methylisothiazolinone | --- | 0,10 | --- | 0,06 | --- |
| Benzethoniumchlorid | 2,0 | --- | --- | 0,15 | --- |
| Piroctonolamin | --- | --- | 0,25 | 0,15 | --- |
| Lauroylethylarginat | --- | --- | --- | 0,15 | 1,75 |
| Glycerin | 5 | 10 | 7,5 | 15 | 7,5 |
| Tocopherolacetat | 0,5 | 0,25 | 0,5 | 0,1 | --- |
| Titandioxid | --- | 2,5 | 1,2 | 0,15 | -- |
| Wasser | ad 100 | ad 100 | ad 100 | ad 100 | ad 100 |

### O/W-Emulsion

| | **31** | **32** | **33** | **34** | **35** |
|---|---|---|---|---|---|
| Polyethylenglycol(21)stearylether | 1 | -- | 2,5 | 2 | 1,5 |
| Polyethylenglycol(2)stearylether | 1 | -- | 5,5 | 3 | 7,5 |
| Cetearylglucosid | --- | 8 | --- | --- | --- |
| Cyclomethicon | 12,5 | 15 | 28,0 | 25,0 | 17,5 |
| Dimethicon | 5,0 | 13,0 | 5,0 | 12,0 | 15,0 |
| Behenylalkohol | 3 | 2 | --- | 1 | --- |
| Stearylalkohol | 3 | 2 | --- | 2 | --- |
| Cetylstearylalkohol | 3 | 4 | --- | --- | 2 |
| Glycerylglucosid | 0,5 | 0,75 | 11,0 | 12,5 | 6,5 |
| Octyldodecanol | 0,5 | 1,0 | 0,75 | 3,0 | 0,25 |
| Glycerin | 5 | 10 | 15 | 3 | 7,5 |
| Methylisothiazolinone | --- | 0,10 | --- | 0,06 | --- |
| Benzethoniumchlorid | 2,0 | --- | --- | 0,15 | --- |
| Piroctonolamin | --- | --- | 0,25 | 0,15 | --- |
| Lauroylethylarginat | --- | --- | --- | 0,15 | 1,75 |
| Panthenol | 0,5 | 1,0 | 0,75 | 0,25 | 0,1 |
| Parfum | q,s, | q,s, | q,s, | q,s, | q,s, |
| modifizierte Stärke | 0,5 | --- | --- | 0,15 | -- |
| Wasser | ad 100 | ad 100 | ad 100 | ad 100 | ad 100 |

### O/W-Emulsion

| | **36** | **37** | **38** | **39** | **40** |
|---|---|---|---|---|---|
| Glycerylsteratcitrat | 1,0 | 0,5 | 0,1 | 0,5 | 0,3 |
| Polyethylenglycol(20)cetearylether | 10,0 | 1,0 | 5 | -- | -- |
| Triglycerinmethylglucosedistearat | --- | --- | --- | --- | 2,5 |
| Cyclomethicon | -- | --- | ---- | 1 | -- |
| Dimethicon | 0,5 | 3,0 | 0,75 | 1,5 | 0,2 |
| Behenylalkohol | 1 | --- | 2 | 1 | 0,2 |
| Glycerylglucosid | 3 | 5 | 10 | 15 | 5 |
| Stearylalkohol | --- | --- | --- | 1 | 0,2 |
| Cetylstearylalkohol | --- | --- | 1 | 1 | 0,2 |
| Tocopherol | 0,5 | 0,5 | 0,75 | 0,25 | 0,1 |
| Octyldodecanol | 0,5 | --- | 0,75 | 3,0 | 0,25 |
| Panthenol | 0,5 | --- | 0,75 | 0,25 | 0,1 |
| Carbomer | 0,05 | 0,35 | 0,15 | 0,1 | --- |
| Parfum | q,s, | q,s, | q,s, | q,s, | q,s, |
| Methylisothiazolinone | --- | 0,10 | --- | 0,06 | --- |
| Benzethoniumchlorid | 2,0 | --- | --- | 0,15 | --- |
| Piroctonolamin | --- | --- | 0,25 | 0,15 | --- |
| Lauroylethylarginat | --- | --- | --- | 0,15 | 1,75 |
| Sorbitol | 10 | --- | -- | 5 | --- |
| Butylenglykol | --- | --- | --- | 5 | 10 |
| Propylenglykol | --- | --- | 10 | 5 | --- |
| Glycerin | --- | 7,5 | --- | --- | --- |
| Wasser | ad 100 | ad 100 | ad 100 | ad 100 | ad 100 |

### O/W-Emulsion

| | **41** | **42** | **43** | **44** | **45** |
|---|---|---|---|---|---|
| Glycerylsteratcitrat | 1,0 | 0,75 | 0,1 | 0,5 | 0,3 |
| Polyethylenglycol(20)cetearylether | 1,0 | --- | 2 | 1,5 | -- |
| Triglycerinmethylglucosedistearat | --- | 5,5 | --- | --- | 2,5 |
| Glycerylglucosid | 5 | 10 | 15 | 20 | 7,5 |
| Cyclomethicon | 2 | 4 | 6 | 1 | 3 |
| Dimethicon | -- | 0,5 | 0,75 | --- | --- |
| Behenylalkohol | 1 | --- | 2 | 1 | 0,2 |
| Stearylalkohol | --- | 1 | --- | 1 | 0,2 |
| Cetylstearylalkohol | --- | --- | 1 | 1 | 0,2 |
| medizinisches Weissöl | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| ^{r} Carbomer | 0,05 | 0,1 | 0,15 | 0,1 | --- |
| Parfum | q,s, | q,s, | q,s, | q,s, | q,s, |
| Dicaprylylcarbonat | 3 | 5 | 10 | 15 | 5 |
| Methylisothiazolinone | --- | 0,10 | --- | 0,06 | --- |
| Benzethoniumchlorid | 2,0 | --- | --- | 0,15 | --- |
| Piroctonolamin | --- | --- | 0,25 | 0,15 | --- |
| Lauroylethylarginat | --- | --- | --- | 0,15 | 1,75 |
| Tocopherol | 0,5 | 1,0 | 0,75 | 0,25 | 0,1 |
| Caprylic/Capric Triglycerid | 1 | 2 | 3 | 5 | 10 |
| modifizierte Stärke | --- | 2,5 | --- | 0,15 | --- |
| Glycerin | 3 | 5 | 8 | 12 | 10 |
| Wasser | ad 100 | ad 100 | ad 100 | ad 100 | ad 100 |

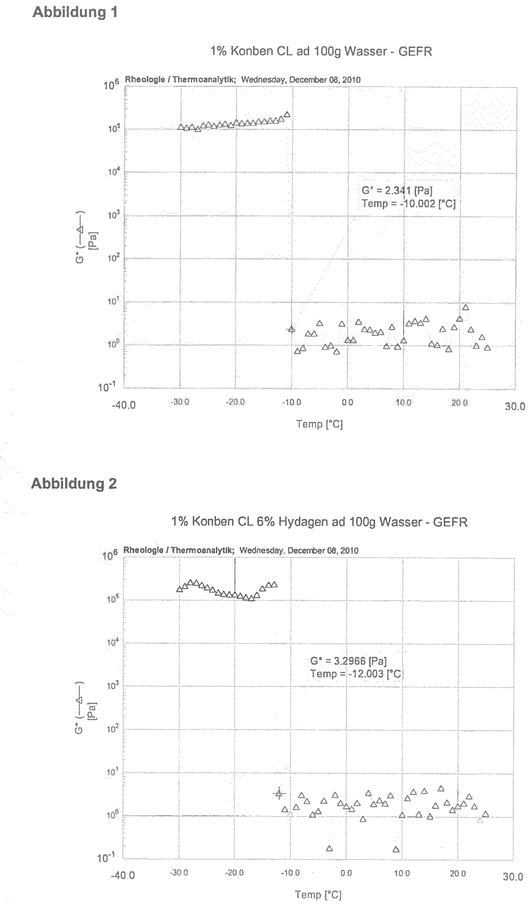

## Patentansprüche

1. Verwendung kosmetischer Zubereitungen, umfassend eine Wasser und/oder Ölphase sowie ferner Wirkstoffkombinationen aus
(i) einem oder mehreren Konservierungsmitteln gewählt aus der Gruppe der Isothiazolinone, Benzethoniumchlorid, Piroctonolamin und Lauroylethylarginat
und.
(ii) einem oder mehreren Glucosylglyceriden gewählt aus der Gruppe der der allgemeinen Formel 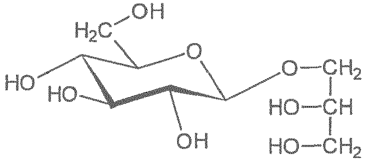 und/oder der allgemeinen Formel 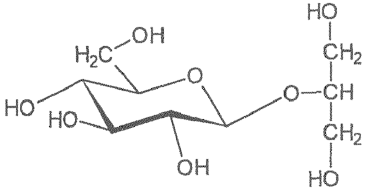 und/oder der allgemeinen Formel 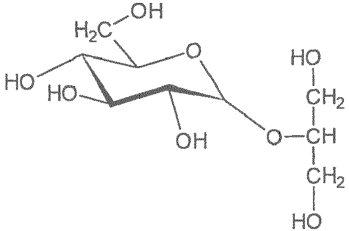 und/oder der allgemeinen Formel 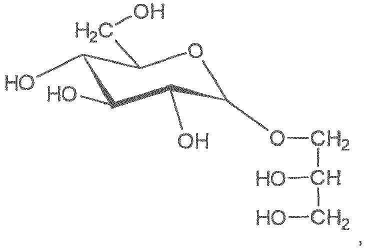 bei denen das oder die Glucosylglyceride in der Wasser- und/oder Ölphase in Konzentrationen von 0,001 - 40,00 Gew.-%, bevorzugt 0,005 - 15,00 Gew.-%, besonders bevorzugt 0,01 - 12,00 Gew.-%, jeweils bezogen auf das Gesamtgewicht der Zusammensetzung, vorliegen und bei denen das oder die mehreren Konservierungsmittel gewählt aus der Gruppe der Isothiazolinone, Benzethoniumchlorid, Piroctonolamin und Lauroylethylarginat in Konzentrationen von 0,00001 bis 10 Gew.-%, bes. bevorzugt 0,001-5 Gew.-%, bes. bevorzugt 0,005% - 3 Gew.-% jeweils bezogen auf das Gesamtgewicht der Zusammensetzung, vorliegt oder vorliegen, ferner enthaltend
(iii) eine Substanz, gewählt aus der Gruppe der anionischen, nichtionischen bzw. amphoteren Tenside und der kationischen Polymere
als Reinigungsmittel.

2. Verwendung nach Anspruch 1, **dadurch gekennzeichnet, daß** sie anionische Tenside in Konzentrationen von 1 bis 20 Gewichts-%, bevorzugt 5 bis 12 Gewichts-%, jeweils bezogen auf das Gesamtgewicht der Zubereitung, enthalten.

3. Verwendung nach Anspruch 1, **dadurch gekennzeichnet, daß** sie amphotere Tenside in Konzentrationen von 1 bis 20 Gewichts-%, bevorzugt 3 bis 8 Gewichts-%, jeweils bezogen auf das Gesamtgewicht der Zubereitung, enthalten.

4. Verwendung nach Anspruch 1, **dadurch gekennzeichnet, daß** sie kationische Polymere in Konzentrationen von 0,01 bis 2 Gewichts-%, bevorzugt 0,05 bis 1,5, Gewichts-%, besonders bevorzugt von 0,1 bis 1 Gew.-% jeweils bezogen auf das Gesamtgewicht der Zubereitung, enthalten.

## Claims

1. Use of cosmetic preparations comprising a water and/or oil phase and further active ingredient combinations of
(i) one or more preservatives selected from the group of isothiazolinones, benzethonium chloride, piroctone olamine and ethyl lauroyl arginate and
(ii) one or more glucosyl glycerides selected from the group of the general formula 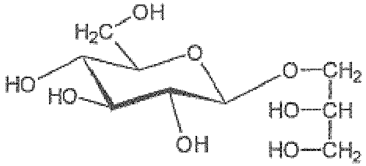 and/or the general formula 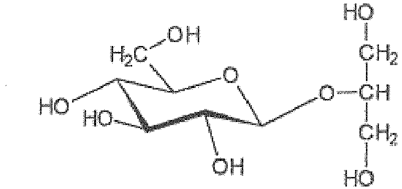 and/or the general formula 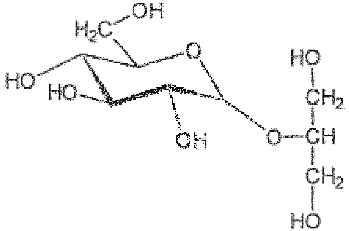 and/or the general formula 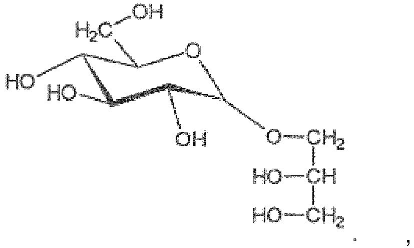 in which the glucosyl glyceride(s) are present in the water and/or oil phase at concentrations of 0.001 - 40.00% by weight, preferably 0.005 - 15.00% by weight, particularly preferably 0.01 - 12.00% by weight, based in each case on the total weight of the composition, and in which the one or more preservative(s) selected from the group of isothiazolinones, benzethonium chloride, piroctone olamine and ethyl lauroyl arginate is/are present at concentrations of 0.00001 to 10% by weight, particularly preferably 0.001-5% by weight, particularly preferably 0.005% - 3% by weight, based in each case on the total weight of the composition, further comprising
(iii) a substance selected from the group of anionic, non-ionic and amphoteric surfactants and cationic polymers
as cleansing agents.

2. Use according to Claim 1, **characterized in that** said preparations comprise anionic surfactants at concentrations of 1 to 20% by weight, preferably 5 to 12% by weight, based in each case on the total weight of the preparation.

3. Use according to Claim 1, **characterized in that** said preparations comprise amphoteric surfactants at concentrations of 1 to 20% by weight, preferably 3 to 8% by weight, based in each case on the total weight of the preparation.

4. Use according to Claim 1, **characterized in that** said preparations comprise cationic polymers at concentrations of 0.01 to 2% by weight, preferably 0.05 to 1.5% by weight, particularly preferably 0.1 to 1% by weight, based in each case on the total weight of the preparation.

## Revendications

1. Utilisation de préparations cosmétiques, comprenant une phase aqueuse et/ou une phase huileuse ainsi qu'en outre des combinaisons de substances actives constituées par
(i) un ou plusieurs conservateurs, choisis dans le groupe formé par les isothiazolinones, le chlorure de benzéthonium, la piroctonolamine et l'arginate de lauroyléthyle et
(ii) un ou plusieurs glucosylglycérides choisis dans le groupe de formule générale 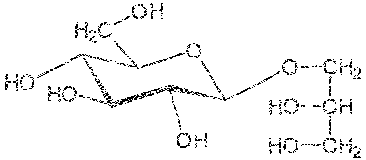 et/ou de formule générale 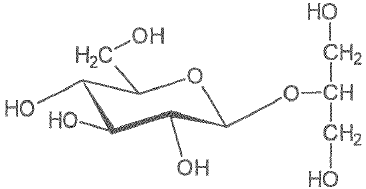 et/ou de formule générale 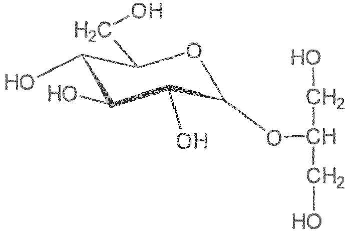 et/ou de formule générale 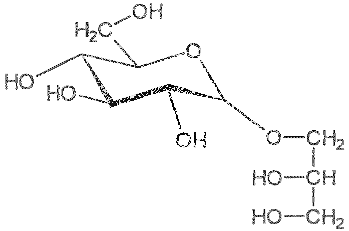 dans lesquelles le ou les glucosylglycérides se trouvent dans la phase aqueuse et/ou dans la phase huileuse en des concentrations de 0,001-40,00% en poids, de préférence de 0,005-15,00% en poids, de manière particulièrement préférée de 0,01-12,00% en poids, à chaque fois par rapport au poids total de la composition et dans lesquelles ledit un ou lesdits plusieurs conservateurs choisis dans le groupe formé par les isothiazolinones, le chlorure de benzéthonium, la piroctonolamine et l'arginate de lauroyléthyle se trouvent en des concentrations de 0,00001 à 10% en poids, de manière particulièrement préférée de 0,001-5% en poids, de manière particulièrement préférée de 0,005%-3% en poids, à chaque fois par rapport au poids total de la composition, contenant en outre
(iii) une substance choisie dans le groupe des agents tensioactifs anioniques, non ioniques ou amphotères et des polymères cationiques
comme agent de nettoyage.

2. Utilisation selon la revendication 1, **caractérisée en ce que** les préparations contiennent des tensioactifs anioniques en des concentrations de 1 à 20% en poids, de préférence de 5 à 12% en poids, à chaque fois par rapport au poids total de la préparation.

3. Utilisation selon la revendication 1, **caractérisée en ce que** les préparations contiennent des tensioactifs amphotères en des concentrations de 1 à 20% en poids, de préférence de 3 à 8% en poids, à chaque fois par rapport au poids total de la préparation.

4. Utilisation selon la revendication 1, **caractérisée en ce que** les préparations contiennent des polymères cationiques en des concentrations de 0,01 à 2% en poids, de préférence de 0,05 à 1,5% en poids, de manière particulièrement préférée de 0,1 à 1% en poids, à chaque fois par rapport au poids total de la préparation.
